# EUROPÄISCHE PATENTSCHRIFT

(11) **EP 3 474 781 B1**
(45) Veröffentlichungstag und Bekanntmachung des Hinweises auf die Patenterteilung: **04.08.2021**
(21) Anmeldenummer: 17733409.1
(22) Anmeldetag: 20.06.2017
(51) Int. Cl.: A61F 2/28, A61L 27/02, A61L 27/04, A61L 27/06, A61L 27/10

(54) **MEDIZINISCHES PRODUKT, VORZUGSWEISE ZUR ANWENDUNG BEI DER BEHANDLUNG EINER KNOCHENKAVITÄT, VERFAHREN ZUR HERSTELLUNG DES MEDIZINISCHEN PRODUKTS SOWIE MEDIZINISCHES KIT**
MEDICAL PRODUCT, PREFERABLY FOR USE DURING THE TREATMENT OF A BONE CAVITY, METHOD FOR PRODUCING THE MEDICAL PRODUCT, AND MEDICAL KIT
PRODUIT MEDICAL, DE PREFERENCE POUR UTILISATION DANS LE TRAITEMENT D'UNE CAVITE OSSEUSE, PROCEDE DE FABRICATION DU PRODUIT MEDICAL ET KIT MEDICALE

(30) Priorität: 22.06.2016 DE 102016211201
(43) Veröffentlichungstag der Anmeldung: 01.05.2019
(73) Patentinhaber: Aesculap AG, 78532 Tuttlingen (DE)
(72) Erfinder: MATTES, Ursula, 78603 Renquishausen (DE); UTZ, Michael, 78532 Tuttlingen (DE)
(74) Vertreter: Patentanwälte Ruff, Wilhelm, Beier, Dauster & Partner mbB
(86) Internationale Anmeldenummer: PCT/EP2017/065144
(87) Internationale Veröffentlichungsnummer: WO 2017/220617

(56) Entgegenhaltungen:
- WO-A1-01/12106
- WO-A1-02/087475
- WO-A1-2011/056422
- US-A1- 2010 010 513
- US-A1- 2015 039 097

## Beschreibung

### ANWENDUNGSGEBIET UND STAND DER TECHNIK

Die Erfindung betrifft ein medizinisches Produkt sowie ein medizinisches Kit, welche sich jeweils insbesondere zur Anwendung bei der Behandlung einer Knochenkavität eignen.

Vor allem bei Revisionen nach einer totalen Hüft- oder Kniearthroplastik besteht häufig der Bedarf, kavitäre Knochendefekte aufzufüllen. Die Auffüllung von Knochendefekten ist teilweise auch im Bereich der Wirbelsäulen- und Traumachirurgie erforderlich.

Insbesondere bei osteoporotischen und tumorbefallenen Knochen gestaltet sich die Auffüllung von kavitären Knochendefekten jedoch häufig schwierig.

Zur intraoperativen Auffüllung von Knochenkavitäten stehen heute mehrere Behandlungsoptionen zur Verfügung.

Eine Behandlungsoption besteht in der Verwendung von patienteneigenem Knochen. Alternativ können Knochen aus einer Knochenbank verwendet werden. Stehen beide Optionen nicht zur Verfügung, muss entweder ein metallisches Knochenersatzmaterial oder ein zementartiges Knochenersatzmaterial, wie beispielsweise Calciumphosphat, Hydroxylapatit oder dergleichen, herangezogen werden. Diese Materialien liegen in der Regel entweder als hartes, gepresstes Formteil oder als loses Pulver vor. Die Adaption dieser festen, künstlichen Knochenersatzmaterialien ist intraoperativ sehr schwierig bis unmöglich, so dass häufig eine vollständige Auffüllung von Knochenkavitäten nicht realisiert werden kann und Hohlräume in den Kavitäten verbleiben. Ein weiterer Nachteil besteht darin, dass gattungsgemäße Knochenersatzmaterialien häufig nur unzureichend in Knochenkavitäten mit kleinen Zugangsöffnungen implantiert werden können. Pulverförmige Knochenersatzmaterialien lassen sich zwar besser in derartige Kavitäten einbringen. Allerdings besitzen pulverförmige Knochenersatzmaterialien den Nachteil, dass sie nur über begrenzt lasttragende Eigenschaften verfügen.

Aus der US 2015/0039097 A1 ist ein als Knochenersatzmaterial verwendbares medizinisches Produkt mit zwei unterschiedlichen Gruppen von Strukturelementen bekannt, wobei sich die Gruppen in Bezug auf die Härte der Strukturelemente voneinander unterscheiden.

Gegenstand der WO 02/087475 A1 ist ein Knochenimplantat mit einer niedrigporösen harten Region und einer hochporösen weichen Region.

Aus der WO 01/12106 A1 sind orthopädische Implantate mit einem äußeren harten Implantatbereich und einem inneren porösen Implantatbereich bekannt.

### AUFGABE UND LÖSUNG

Der vorliegenden Erfindung liegt daher die Aufgabe zugrunde, ein medizinisches Produkt sowie ein Verfahren zur Herstellung eines medizinischen Produkts bereitzustellen, wobei sich das Produkt vor allem zur Behandlung von Knochenkavitäten eignen und insbesondere Nachteile von aus dem Stand der Technik bekannten Knochenersatzimplantaten sowie -materialien möglichst weitgehend vermeiden soll.

Weiterhin liegt der Erfindung die Aufgabe zugrunde, ein medizinisches Kit bereitzustellen, welches vor allem zur Behandlung von Knochenkavitäten einsetzbar ist.

Diese Aufgabe wird gelöst durch ein medizinisches Produkt gemäß unabhängigem Anspruch 1. Bevorzugte Ausführungsformen sind in den abhängigen Ansprüchen definiert. Der Wortlaut sämtlicher Ansprüche wird hiermit durch ausdrückliche Bezugnahme zum Inhalt der vorliegenden Beschreibung gemacht.

Gemäß einem ersten Aspekt betrifft die Erfindung ein medizinisches Produkt, vorzugsweise zur Anwendung bei der Behandlung, insbesondere zur Anwendung beim Auffüllen und/oder beim Verschluss, einer Knochenkavität.

Das medizinische Produkt weist miteinander verbundene Strukturelemente auf. Die Strukturelemente sind in wenigstens zwei Gruppen von Strukturelementen einteilbar, nämlich wenigstens in eine erste Gruppe von Strukturelementen und in eine zweite Gruppe von Strukturelementen.

Das medizinische Produkt zeichnet sich dadurch aus, dass die Strukturelemente der ersten Gruppe eine niedrigere Härte aufweisen als die Strukturelemente der zweiten Gruppe. Anders ausgedrückt, zeichnet sich das medizinische Produkt dadurch aus, dass es Strukturelemente mit unterschiedlichen Härten aufweist, nämlich wenigstens eine erste Gruppe von Strukturelemente und eine zweite Gruppe von Strukturelementen, wobei die Strukturelemente der ersten Gruppe eine niedrigere Härte aufweisen als die Strukturelemente der zweiten Gruppe.

Unterschiedliche Härten der Strukturelemente bewirken insbesondere Unterschiede in den Festigkeiten, insbesondere Zug-, Druck- und/oder Biegefestigkeiten, der Strukturelemente. Dies ermöglicht mit besonderem Vorteil eine bessere, insbesondere variablere, Anpassung des medizinischen Produkts an spezifische Gegebenheiten wie insbesondere Ausdehnung bzw. Form und Zugangsöffnung einer Knochenkavität. Gleichzeitig können für eine nachhaltige Behandlung einer Knochenkavität erforderliche mechanische Eigenschaften des medizinischen Produkts über die Ausgestaltung der Strukturelemente, insbesondere über die Auswahl von Materialien zu deren Herstellung, gezielt beeinflusst bzw. gesteuert werden.

Die hergestellten Produkte haben insbesondere den Vorteil, dass sie aufgrund der anwesenden flexiblen Strukturelemente die Tendenz haben, sich in einer Knochenkavität auszubreiten. Hierdurch kommt es zu einem Auffüllen der Knochenkavität. Dabei stellen sich die einzelnen Strukturelemente gegeneinander auf und bilden so ein stabiles, lasttragendes Gebilde. Die lasttragenden Eigenschaften des Gebildes rühren dabei hauptsächlich von den Strukturelementen der zweiten Gruppe. Durch ein entsprechendes Positionieren der Produkte lässt sich die Öffnung einer Knochenkavität auch verschließen, da sich das Produkt von der Innenseite der Kavität über die Öffnung legen kann.

Unter dem Ausdruck "Härte" soll im Sinne der vorliegenden Erfindung der mechanische Widerstand verstanden werden, welchen ein Werkstoff der mechanischen Eindringung eines anderen Körpers (Eindringkörper) entgegensetzt (sogenannte Eindringhärte). Die Härte berechnet sich im Allgemeinen durch den Quotienten aus Prüfkraft (F) und Eindruckfläche (A).

Die Härte von Kunststoffen oder Polymeren, insbesondere Elastomeren, kann beispielsweise gemäß den Normen DIN EN ISO 868 und DIN ISO 7619-1 bestimmt werden. Hierbei wird ein federbelasteter Stift aus gehärtetem Stahl eines Shore-Härte-Prüfgeräts in einen zu prüfenden Werkstoff eingedrückt. Die Eindringtiefe des Stifts in den zu prüfenden Werkstoff ist ein Maß für die Shore-Härte, welche auf einer Skala von 0 Shore (2.5 mm Eindringtiefe) bis 100 Shore (0 mm Eindringtiefe) gemessen wird. Eine hohe Zahl bedeutet also eine große Härte. Bei dem Shore-Härte-Prüfgerät ist eine Zusatzeinrichtung einsetzbar, welche die zu messende Probe mit einer Kraft von 12.5 N bei Shore-A bzw. 50 N bei Shore-D auf den Messtisch andrückt. Die Solltemperatur zur Messung der Shore-Härte beträgt in der Regel 23°C und wird vorzugsweise auf das Temperaturintervall von +/- 2 K beschränkt.

Shore-A wird insbesondere bei Weichelastomeren angegeben. Als Eindringkörper wird eine Nadel mit abgestumpfter Spitze verwendet. Die Stirnfläche des Kegelstumpfs hat einen Durchmesser von 0.79 mm, der Öffnungswinkel beträgt 35°. Das Auflagegewicht beträgt 1 kg und die Haltezeit 15 s.

Shore-D wird insbesondere angegeben bei Zäh-Elastomeren nach Messung mit einer Nadel, welche mit einem 30°-Winkel zuläuft und eine kugelförmige Spitze mit einem Durchmesser von 0.2 mm hat. Das Auflagegewicht beträgt 5 kg und die Haltezeit ebenfalls 15 s.

Die Härte von homogenen, dünnwandigen oder oberflächengehärteten Werkstoffen kann beispielsweise mittels Härteprüfung nach Vickers (HV) bestimmt werden. Hierbei wird eine gleichseitige Diamantpyramide mit einem Öffnungswinkel von 136° unter einer festgelegten Prüfkraft in das Werkstück eingedrückt. Aus der mittels eines Messmikroskops festgestellten Länge der Diagonalen des bleibenden Eindrucks wird die Eindruckoberfläche errechnet. Das Verhältnis von Prüfkraft in der Einheit N zur Eindruckoberfläche d (in mm) ergibt mit dem Faktor 0.1891 multipliziert die Vickers-Härte (HV, engl. VHN = Vickers Hardness Number). Die Prüfkraft F kann 0.098 N ≤ F < 1.961 N (Vickers-Mikrohärteprüfung), 1.961 N ≤ F < 49.03 N (Vickers-Kleinkrafthärteprüfung) oder F ≥ 49.03 N (Vickers-Härteprüfung) betragen.

Die Härte von Metallen kann beispielsweise mittels instrumentierter Eindringprüfung gemäß DIN EN ISO 14577 gemessen werden. Hierbei wird ein Eindringkörper spezieller Geometrie, beispielsweise ein pyramidenförmiger Eindringkörper (Vickerspyramide), in einen zu prüfenden Werkstoff eingedrückt. Prüfkraft und Eindringtiefe werden simultan gemessen. Je härter der Werkstoff ist, desto geringer ist die Eindringtiefe des Eindringkörpers.

In Übereinstimmung mit den vorangegangenen Ausführungen setzen bei der vorliegenden Erfindung die Strukturelemente der ersten Gruppe dem Eindringen eines Eindringkörpers (wie beispielsweise eines Stifts, einer Nadel oder einer Pyramide) einen kleineren mechanischen Widerstand entgegen als die Strukturelemente der zweiten Gruppe dem Eindringen desselben Eindringkörpers. Das Eindringen eines Eindringkörpers in ein Strukturelement der ersten Gruppe führt daher zu einer größeren Eindringtiefe als das Eindringen desselben Eindringkörpers in ein Strukturelement der zweiten Gruppe.

Unter dem Ausdruck "Knochenkavität" soll im Sinne der vorliegenden Erfindung ein Hohlraum in einem menschlichen oder tierischen Knochen, insbesondere menschlichen oder tierischen Gelenksknochen, vorzugsweise menschlichen oder tierischen Hüftgelenks- oder Kniegelenksknochen, oder menschlichen oder tierischen Wirbelkörper, verstanden werden. Der Hohlraum kann die Folge einer Knochenfraktur, eines Knochentraumas, einer Knochenerkrankung, einer Tumorerkrankung oder einer chirurgischen Intervention/Reintervention, insbesondere einer Revision nach einer totalen Hüft- oder Kniearthroplastik, sein.

In einer besonders bevorzugten Ausführungsform handelt es sich bei dem medizinischen Produkt um ein Implantat, vorzugsweise um ein Knochenersatzmaterial oder Knochenfüllmaterial.

Vorzugsweise sorgen in erster Linie die Strukturelemente der ersten Gruppe für eine gewünschte Anpassungsfähigkeit des medizinischen Produkts an Knochenkavitäten. Es ist daher bevorzugt, wenn die Strukturelemente der ersten Gruppe flexibel bzw. biegsam, insbesondere elastisch, ausgebildet sind. Die Strukturelemente der ersten Gruppe können im Sinne der vorliegenden Erfindung daher auch als flexible Strukturelemente bezeichnet werden. Besonders bevorzugt sind die Strukturelemente der ersten Gruppe flexibler bzw. biegsamer, insbesondere elastischer, ausgebildet als die Strukturelemente der zweiten Gruppe. Dadurch ist eine besonders wirkungsvolle Anpassung des medizinischen Produkts an eine Knochenkavität, insbesondere an eine Zugangsöffnung davon, erzielbar.

Demgegenüber sind die Strukturelemente der zweiten Gruppe vorzugsweise in erster Linie für die mechanische Stabilität wie Formstabilität des medizinischen Produkts verantwortlich. Es ist daher bevorzugt, wenn die Strukturelemente der zweiten Gruppe lasttragend bzw. formstabil ausgebildet sind. Die Strukturelemente der zweiten Gruppe können im Sinne der vorliegenden Erfindung daher auch als lasttragende bzw. formstabile Strukturelemente bezeichnet werden. Besonders bevorzugt sind die Strukturelemente der zweiten Gruppe lasttragender bzw. formstabiler ausgebildet als die Strukturelemente der ersten Gruppe. Dadurch kann mit besonderem Vorteil gewährleistet werden, dass das medizinische Produkt über eine ausreichend mechanische Stabilität verfügt, um eine Knochenkavität dauerhaft behandeln, insbesondere auffüllen und/oder verschließen, zu können.

In einer weiteren Ausführungsform ist das medizinische Produkt begrenzt verformbar. Bei dieser Ausführungsform geht eine Verformbarkeit des medizinischen Produkts vorzugsweise auf die Strukturelemente der ersten Gruppe zurück, während vorzugsweise die Strukturelemente der zweiten Gruppe eine Verformbarkeit des Produkts begrenzen.

In einer weiteren Ausführungsform ist das medizinische Produkt reversibel verformbar. Bei dieser Ausführungsform sind vorzugsweise elastisch ausgebildete Strukturelemente der ersten Gruppe für eine reversible Verformbarkeit des medizinischen Produkts verantwortlich, während vorzugsweise die Strukturelemente der zweiten Gruppe eine reversible Verformbarkeit des Produkts begrenzen.

In einer weiteren Ausführungsform weisen die Strukturelemente der ersten Gruppe jeweils eine Härte (Shore D) von 30 bis 100, insbesondere 35 bis 80, bevorzugt 40 bis 60 auf.

Für den Fall, dass die Strukturelemente der zweiten Gruppe ein Metall aufweisen oder aus einem Metall bestehen, weisen die Strukturelemente der zweiten Gruppe in einer weiteren Ausführungsform jeweils bei einer Prüfkraft von 10 N eine Härte nach Vickers von 100 bis 1700, insbesondere 150 bis 800, vorzugsweise 200 bis 600, auf.

Für den Fall, das die Strukturelemente der zweiten Gruppe einen oberflächengehärteten Kunststoff, insbesondere einen oberflächengehärteten Kunststoff mit einer Beschichtung, aufweisen oder aus einem solchen Kunststoff bestehen, weisen die Strukturelemente der zweiten Gruppe in einer weiteren Ausführungsform jeweils bei einer Prüflast bzw. Prüfmasse von 0.025 g eine Härte nach Vickers von 100 bis 1700, insbesondere 150 bis 800, vorzugsweise 200 bis 600, auf.

In einer weiteren Ausführungsform weisen die Strukturelemente der ersten Gruppe eine niedrigere Festigkeit, insbesondere eine niedrigere Zugfestigkeit, eine niedrigere Druckfestigkeit oder eine niedrigere Biegefestigkeit wie niedrigere Biegezugfestigkeit, auf als die Strukturelemente der zweiten Gruppe.

Die Strukturelemente der ersten Gruppe können insbesondere eine Zugfestigkeit von 1 N/mm² bis 30 N/mm², insbesondere 5 N/mm² bis 28 N/mm², vorzugsweise 7 N/mm² bis 25 N/mm², aufweisen.

Die Strukturelemente der zweiten Gruppe können insbesondere eine Zugfestigkeit von 50 N/mm² bis 1100 N/mm², insbesondere 60 N/mm² bis 900 N/mm², vorzugsweise 70 N/mm² bis 900 N/mm², aufweisen.

Weiterhin können die Strukturelemente der ersten Gruppe insbesondere eine Druckfestigkeit von 5 N/mm² bis 150 N/mm², insbesondere 10 N/mm² bis 100 N/mm², vorzugsweise 12 N/mm² bis 90 N/mm², aufweisen.

Die Strukturelemente der zweiten Gruppe können weiterhin insbesondere eine Druckfestigkeit von 100 N/mm² bis 3000 N/mm², insbesondere 150 N/mm² bis 2000 N/mm², vorzugsweise 150 N/mm² bis 1500 N/mm², aufweisen.

Des Weiteren können die Strukturelemente der ersten Gruppe insbesondere eine Biegefestigkeit wie Biegezugfestigkeit von 3 N/mm² bis 100 N/mm², insbesondere 5 N/mm² bis 80 N/mm², vorzugsweise 10 N/mm² bis 50 N/mm², aufweisen.

Die Strukturelemente der zweiten Gruppe können des Weiteren insbesondere eine Biegefestigkeit wie Biegezugfestigkeit von 30 N/mm² bis 630 N/mm², insbesondere 40 N/mm² bis 500 N/mm², vorzugsweise 50 N/mm² bis 400 N/mm², aufweisen.

In einer weiteren Ausführungsform sind die Strukturelemente der ersten Gruppe gleich ausgebildet.

In einer weiteren Ausführungsform sind die Strukturelemente der ersten Gruppe in zwei oder mehr Untergruppen einteilbar, wobei die Strukturelemente jeder Untergruppe identisch zueinander und die Strukturelemente unterschiedlicher Untergruppen unterschiedlich zueinander ausgebildet sind. Beispielsweise können die Strukturelemente unterschiedlicher Untergruppen in Bezug auf ihre Härte, Festigkeit (wie Zug-, Druck- und/oder Biegefestigkeit), ihre Form bzw. Gestalt, ihre Abmessungen (wie beispielsweise Länge, Breite, Dicke bzw. Höhe, Radius und/oder Durchmesser) und/oder ihres Materials unterschiedlich zueinander ausgebildet sein.

In einer weiteren Ausführungsform sind die Strukturelemente der zweiten Gruppe gleich ausgebildet.

In einer weiteren Ausführungsform sind die Strukturelemente der zweiten Gruppe in zwei oder mehr Untergruppen einteilbar, wobei die Strukturelemente jeder Untergruppe identisch zueinander und die Strukturelemente unterschiedlicher Untergruppen unterschiedlich zueinander ausgebildet sind. Beispielsweise können die Strukturelemente unterschiedlicher Untergruppen in Bezug auf ihre Härte, Festigkeit (wie Zug-, Druck- und/oder Biegefestigkeit), ihre Form bzw. Gestalt, ihre Abmessungen (wie beispielsweise Länge, Breite, Dicke bzw. Höhe, Radius und/oder Durchmesser) und/oder ihres Materials unterschiedlich zueinander ausgebildet sein.

In einer weiteren Ausführungsform handelt es sich bei den Strukturelementen der ersten Gruppe jeweils um längliche Strukturelemente, d.h. um Strukturelemente mit einem Länge-Breite-Verhältnis oder Länge-Durchmesser-Verhältnis > (gesprochen: größer) 1. Dadurch ist mit besonderem Vorteil eine wenigstens abschnittsweise Einfassung (Bordierung) oder Umrandung der Strukturelemente der zweiten Gruppe durch die Strukturelemente der ersten Gruppe möglich. Die Strukturelemente der ersten Gruppe können insbesondere stegförmig ausgebildet sein.

In einer weiteren Ausführungsform weisen die Strukturelemente der ersten Gruppe ein Dicke-Breite-Verhältnis (Höhe-Breite-Verhältnis) > (gesprochen: größer) oder < (gesprochen: kleiner) 1 auf. Durch derartige Verhältnisse können die Flexibilität und/oder Stabilität des medizinischen Produkts gezielt beeinflusst und insbesondere an eine zu versorgende Knochenkavität angepasst werden.

In einer weiteren Ausführungsform weisen die Strukturelemente der ersten Gruppe einen über die Strukturelementlänge variablen Querschnitt auf. Der Querschnitt kann dabei hinsichtlich seiner Abmessungen und/oder seiner Umrissform entlang der Strukturelementlänge variieren. Beispielsweise können die Strukturelemente der ersten Gruppe kegelstumpfförmig ausgebildet sein.

In einer weiteren Ausführungsform sind die Strukturelemente der zweiten Gruppe jeweils in Form von Zylindern, insbesondere Flachzylindern, mit kreisförmigen oder polygonen Stirnflächen ausgebildet. Bei den polygonen Stirnflächen kann es sich insbesondere um dreieckförmige, viereckförmige wie quadrat- oder rechteckförmige, fünfeckförmige, sechseckförmige oder um Kombinationen aus zwei verschiedenen der genannten Stirnflächen handeln.

Grundsätzlich können die Strukturelemente der ersten Gruppe und die Strukturelemente der zweiten Gruppe jeweils die gleiche Breite oder den gleichen Durchmesser besitzen.

Alternativ können die Strukturelemente der ersten Gruppe jeweils eine kleinere Breite oder einen kleineren Durchmesser aufweisen als die Strukturelemente der zweiten Gruppe oder umgekehrt, d.h. die Strukturelemente der zweiten Gruppe können jeweils eine kleinere Breite oder einen kleineren Durchmesser aufweisen als die Strukturelemente der ersten Gruppe.

In einer weiteren Ausführungsform weisen die Strukturelemente der ersten Gruppe jeweils eine geringere Masse auf als die Strukturelemente der zweiten Gruppe. Dadurch ist eine zusätzliche Verbesserung der Anpassungsfähigkeit des medizinischen Produkts an eine Knochenkavität erzielbar.

Grundsätzlich können die Strukturelemente der ersten Gruppe und/oder die Strukturelemente der zweiten Gruppe eine Anzahl von Löchern, d.h. ein Loch oder mehrere Löcher, aufweisen.

Das Loch/die Löcher kann/können als Vertiefung/Vertiefungen und/oder als Öffnung/Öffnungen, d.h. als Durchbruch/Durchbrüche, ausgebildet sein.

Erfindungsgemäß kann es bevorzugt sein, wenn nur die Strukturelemente der ersten Gruppe eine Anzahl von Löchern aufweisen. Alternativ können die Strukturelemente der ersten Gruppe eine größere Anzahl an Löchern und/oder größere Löcher aufweisen als die Strukturelemente der zweiten Gruppe. Dies kann mit besonderem Vorteil zu einer Erhöhung der Flexibilität, insbesondere Biegsamkeit, der Strukturelemente der ersten Gruppe gegenüber den Strukturelementen der zweiten Gruppe beitragen, wodurch die Anpassungsfähigkeit des medizinischen Produkts an eine Knochenkavität zusätzlich verbessert werden kann.

In einer weiteren Ausführungsform weist das medizinische Produkt ein aus den Strukturelementen der ersten und zweiten Gruppen aufgebautes Flächengebilde auf. Bevorzugt sind die Länge und die Breite des Flächengebildes jeweils größer als die Dicke bzw. Höhe des Flächengebildes. Bei dem Flächengebilde handelt es sich vorzugsweise um ein platten- oder teppichförmiges Flächengebilde.

In einer weiteren Ausführungsform weist das medizinische Produkt, insbesondere das Flächengebilde, eine variierende, d.h. nicht konstante, Dicke bzw. Höhe auf. Beispielsweise kann das medizinische Produkt, insbesondere das Flächengebilde, eine variierende Dicke von 0.1 mm bis 10 mm, insbesondere 0.3 mm bis 5 mm, bevorzugt 0.5 mm bis 1.5 mm, aufweisen.

In einer alternativen Ausführungsform weist das medizinische Produkt, insbesondere das Flächengebilde, eine konstante, d.h. gleichbleibende bzw. einheitliche, Dicke auf. Beispielsweise kann das medizinische Produkt, insbesondere das Flächengebilde, eine konstante Dicke von 0.1 mm bis 10 mm, insbesondere 0.3 mm bis 5 mm, bevorzugt 0.5 mm bis 1.5 mm, aufweisen.

In einer unter Befestigungsgesichtspunkten vorteilhaften Ausführungsform weisen Randbereiche, insbesondere Eckbereiche, des medizinischen Produkts, insbesondere des Flächengebildes, eine Anzahl von Befestigungseinrichtungen, d.h. eine Befestigungseinrichtung oder mehrere Befestigungseinrichtungen, oder eine Anzahl von Löchern, d.h. ein Loch oder mehrere Löcher, für eine Befestigungseinrichtung auf. Bei den Löchern kann es sich beispielsweise um Durchbrüche bzw. Öffnungen von Strukturelementen, insbesondere von Strukturelementen der ersten Gruppe, handeln. Derartige Strukturelemente können insbesondere an den Randbereichen, insbesondere Eckbereichen, des medizinischen Produkts, insbesondere des Flächengebildes, ausgebildet sein. Die Durchbrüche bzw. Öffnungen erlauben beispielsweise das Einfädeln von chirurgischen Nahtmaterialien oder das Eingreifen von Knochenschrauben, wodurch eine sichere Befestigung des medizinischen Produkts, insbesondere des Flächengebildes, in einer Knochenkavität möglich ist. Bei den Befestigungseinrichtungen kann es sich demnach beispielsweise um chirurgische Nahtmaterialien oder Knochenschrauben handeln.

In einer weiteren Ausführungsform weist das medizinische Produkt, insbesondere das Flächengebilde, eine Verdickung auf, welche wenigstens abschnittsweise entlang der Kante des medizinischen Produkts, insbesondere des Flächengebildes, ausgebildet ist. Bei der Verdickung kann es sich daher um eine Art Randsteg handeln. Bevorzugt ist die Verdickung eine umlaufende Verdickung, d.h. eine Verdickung, welche durchgehend entlang der Kante des medizinischen Produkts, insbesondere des Flächengebildes, ausgebildet ist. Erfindungsgemäß ist es weiterhin bevorzugt, wenn die Verdickung von Strukturelementen der ersten Gruppe gebildet ist. Die Verdickung kann beispielsweise auf einer breiteren Ausgestaltung der Strukturelemente der ersten Gruppe gegenüber den Strukturelementen der zweiten Gruppe beruhen. Die in diesem Absatz beschriebene Verdickung hat insbesondere den Vorteil, dass sie mit Befestigungseinrichtungen, wie beispielsweise im vorherigen Absatz beschrieben, verbunden oder mit Löchern zur Aufnahme oder zum Eingreifen von Befestigungseinrichtungen versehen werden kann. Außerdem kann durch die Verdickung eine höhere Stabilität der Außenkante des medizinischen Produkts, insbesondere des Flächengebildes, erzielt werden.

Miteinander verbundene Strukturelemente der ersten Gruppe bilden eine Anzahl von Sollbiegebereichen, d.h. einen Sollbiegebereich oder eine Mehrzahl von Sollbiegebereichen, beispielsweise zwei, drei, vier, fünf, sechs, sieben, acht, neun, zehn, elf, zwölf oder oder mehr Sollbiegebereiche des medizinischen Produkts, insbesondere des Flächengebildes.

Der nachfolgend verwendete Pluralausdruck "Sollbiegebereiche" kann entsprechend der im vorherigen Absatz zum Ausdruck "Anzahl von Sollbiegebereichen" gemachten Ausführung einen Sollbiegebereich (Singular) oder eine Mehrzahl von Sollbiegebereichen (Plural) bedeuten. Bevorzugt bedeutet der nachfolgend verwendete Pluralausruck "Sollbiegebereiche jedoch eine Mehrzahl von Sollbiegebereichen.

Vorzugsweise umranden die Sollbiegebereiche jeweils wenigstens ein, insbesondere nur ein, Strukturelement der zweiten Gruppe. Mit anderen Worten ist es erfindungsgemäß bevorzugt, wenn die Sollbiegebereiche jeweils eine Umrandung für wenigstens ein, insbesondere nur ein, Strukturelement der zweiten Gruppe bilden.

Die Sollbiegebereiche können das wenigstens eine Strukturelement der zweiten Gruppe insbesondere eckenlos, beispielsweise kreisringförmig, umranden.

Alternativ können die Sollbiegebereiche das wenigstens eine Strukturelement der zweiten Gruppe polygon, insbesondere dreieckförmig, viereckförmig wie quadrat- oder rechteckförmig, fünfeckförmig und/oder sechseckförmig, bevorzugt dreieckförmig, viereckförmig wie quadrat- oder rechteckförmig, fünfeckförmig oder sechseckförmig, umranden.

Die Sollbiegebereiche sind weiterhin vorzugsweise reihen- oder linienförmig, insbesondere geradlinig, verlaufend ausgebildet.

Weiterhin können die Sollbiegebereiche nicht geradlinig, insbesondere zickzackförmig, kurvenförmig, bogenförmig oder mäanderförmig wie sinusförmig verlaufend ausgebildet sein.

Die Sollbiegebereiche sind in einer weiteren Ausführungsform in Längsrichtung, Breitenrichtung und/oder Schrägrichtung, insbesondere Diagonalrichtung, des medizinischen Produkts, insbesondere des Flächengebildes, verlaufend ausgebildet.

Weiterhin können die Sollbiegebereiche zueinander parallel verlaufend ausgebildet sein.

Insbesondere können die Sollbiegebereiche in Form von zueinander parallel angeordneten Reihen ausgebildet sein.

Alternativ können die Sollbiegebereiche in einem Winkel, insbesondere orthogonal, d.h. in einem rechten Winkel, zueinander angeordnet sein.

Bei den Sollbiegebereichen handelt es sich vorzugsweise um Faltbereiche des medizinischen Produkts, insbesondere des Flächengebildes.

In einer weiteren Ausführungsform sind die Sollbiegebereiche wenigstens zum Teil über ein Strukturelement der zweiten Gruppe oder über mehrere Strukturelemente der zweiten Gruppe miteinander verbunden.

In einer weiteren Ausführungsform weisen die Strukturelemente der ersten Gruppe ein resorbierbares Material auf oder bestehen aus einem resorbierbaren Material. In einer weiteren Ausführungsform weisen die Strukturelemente der zweiten Gruppe ein resorbierbares Material auf oder bestehen aus einem resorbierbaren Material. Insbesondere können die Strukturelemente der ersten Gruppe und die Strukturelemente der zweiten Gruppe ein resorbierbares Material aufweisen oder aus einem resorbierbaren Material bestehen. Erfindungsgemäß kann es jedoch bevorzugt sein, wenn nur die Strukturelemente der ersten Gruppe ein resorbierbares Material aufweisen oder aus einem resorbierbaren Material bestehen. Die Verwendung eines resorbierbaren Materials für die Strukturelemente der ersten Gruppe und/oder für die Strukturelemente der zweiten Gruppe kann insbesondere für die Ausbildung eines medizinischen Produkts mit osteokonduktiven Eigenschaften von Vorteil sein, da sich ein Resorptionsprozess innerhalb einer Knochenkavität förderlich auf das Einwachsen von Knochengewebe in die Kavität hinein auswirken kann.

Das resorbierbare Material kann insbesondere ausgewählt sein aus der Gruppe bestehend aus Polymer wie Polyhydroxyalkanoat, Elastomer, Protein wie extrazelluläres Protein, Polysaccharid wie Cellulosederivat und/oder Mucopolysaccharid, Knochenzementmaterial, Metall und Kombinationen, insbesondere Verbundstoffe, Mischungen oder Blends, aus wenigstens zwei der genannten Materialien.

Das Polymer, insbesondere Polyhydroxyalkanoat, kann insbesondere ausgewählt sein aus der Gruppe bestehend aus Polymilchsäure bzw. Polylactid, Polyglycolsäure bzw. Polyglycolid, Poly-3-hydroxybutyrat, Poly-4-hydroxybutyrat, Polytrimethylencarbonat, Poly-ε-caprolacton, Stereoisomere, insbesondere Diastereomere, davon, Copolymere davon und Kombinationen, insbesondere Verbundstoffe, Mischungen oder Blends, aus wenigstens zwei der genannten Polymere.

Das Protein kann ausgewählt sein aus der Gruppe bestehend aus Collagen, Gelatine, Elastin, Retikulin, Fibronektin, Laminin, Fibrin, Fibrinogen, Albumin wie Serumalbumin, Salze davon, Stereoisomere, insbesondere Diastereomere, davon und Kombinationen, insbesondere Verbundstoffe, Mischungen oder Blends, aus wenigstens zwei der genannten Proteine.

Das Polysaccharid kann insbesondere ausgewählt sein aus der Gruppe bestehend aus Stärke, Amylose, Amylopektin, Dextran, Dextrin, Cellulose, Alkylcellulose, Hydroxyalkylcellulose, Hydroxymethylcellulose, Hydroxyethylcellulose, Hydroxypropylcellulose, Carboxyalkylcellulose, Carboxymethylcellulose, Chitin, Chitosan, Hyaluronsäure, Dextransulfat, Heparin, Heparansulfat, Chondroitinsulfat, Dermatansulfat, Salze davon, Stereoisomere, insbesondere Diastereomere, davon und Kombinationen, insbesondere Verbundstoffe, Mischungen oder Blends, aus wenigstens zwei der genannten Polysaccharide.

Das Knochenzementmaterial kann insbesondere eine Calcium- und/oder Magnesiumverbindung aufweisen oder aus einer Calcium- und/oder Magnesiumverbindung bestehen, welche ausgewählt ist aus der Gruppe bestehend aus Monocalciumphosphat-Monohydrat (MCPM), Monocalciumphosphat-Anhydrid (MCPA), Dicalciumphosphat, Dicalciumphosphat-Anhydrid (DCPA), Dicalciumphosphat-Dihydrat (DCPD), Octacalciumphosphat (OCP), Tricalciumphosphat, α-Tricalciumphosphat (α-TCP), β-Tricalciumphosphat (β-TCP), amorphes Calciumphosphat (ACP), Hydroxylapatit (HA), Calcium-defizientes Hydroxylapatit (CdHA), substituiertes Hydroxylapatit, nicht stöchiometrisches Hydroxylapatit, nanoskaliges Hydroxylapatit, Tetracalciumphosphat (TTCP), Calciumsulfat (CaSO₄), Calciumsulfat-Hemihydrat (CaSO₄ x 0.5 H₂O), Calciumsulfat-Dihydrat (CaSO₄ x 2 H₂O), Calciumoxid (CaO), Calciumhydroxid (Ca(OH)₂), Calciumcarbonat (CaCO₃), Calciumglycerophosphat, Calciumcitrat, Calciumlactat, Calciumacetat, Calciumtartrat, Calciumchlorid (CaCl₂), Calciumsilicate, Magnesiumhydrogenphosphat (MgH-PO₄) in Form der Hydrate oder als wasserfreie Substanz, Trimagnesiumphosphat (Mg₃(PO₄)₂), Magnesiumdihydrogenphosphat (Mg(H₂PO₄)₂) in Form der Hydrate oder als wasserfreie Substanz, Magnesiumchlorid (MgCl₂) in Form der Hydrate oder als wasserfreie Substanz, Magnesiumglycerophosphat, Magnesiumhydroxid (Mg(OH)₂), Magnesiumhydroxidcarbonat (beispielsweise als 4 MgCO₃ x Mg(OH)₂ x 5 H₂O), Magnesiumoxid (MgO), Magnesiumcitrate (Mg₃(C₆H₅O₇)₂) oder Mg(C₆H₆O₇)), Calcium-Magnesiumcarbonat (CaMg(CO₃)₂, Dolomit und Mischungen aus wenigstens zwei der genannten Verbindungen.

Das Metall kann insbesondere ausgewählt sein aus der Gruppe bestehend aus Magnesium, Tantal und Kombinationen, insbesondere Legierungen, davon.

In einer weiteren Ausführungsform weisen die Strukturelemente der ersten Gruppe ein nicht resorbierbares Material auf oder bestehen aus einem nicht resorbierbaren Material. In einer weiteren Ausführungsform weisen die Strukturelemente der zweiten Gruppe ein nicht resorbierbares Material auf oder bestehen aus einem nicht resorbierbaren Material. Insbesondere können die Strukturelemente der ersten Gruppe und die Strukturelemente der zweiten Gruppe ein nicht resorbierbares Material aufweisen oder aus einem nicht resorbierbaren Material bestehen. Erfindungsgemäß kann es jedoch bevorzugt sein, wenn nur die Strukturelemente der zweiten Gruppe ein nicht resorbierbares Material aufweisen oder aus einem nicht resorbierbaren Material bestehen. Durch die Verwendung eines nicht resorbierbaren Materials für die Strukturelemente der ersten Gruppe und/oder für die Strukturelemente der zweiten Gruppe kann insbesondere ein dauerhaftes Produkt mit (im Wesentlichen) dauerhaft konstant bleibenden mechanischen Eigenschaften ausgebildet werden.

Das nicht resorbierbare Material kann insbesondere ausgewählt sein aus der Gruppe bestehend aus Polymer oder Kunststoff, Elastomer, Thermoplast, Kunststoff, Polyolefin, Polyester, Polyamid, Polyether, Polycarbonat, Silikone, oberflächengehärteter Kunststoff, Keramik, Metall und Kombinationen, insbesondere Verbundstoffe, Mischungen oder Blends, aus wenigstens zwei der genannten Materialien.

Das Polyolefin kann insbesondere ausgewählt sein aus der Gruppe bestehend aus Polyethylen, Polyethylen niedriger Dichte, Polyethylen hoher Dichte, ultrahochmolekulares Polyethylen (UHMWPE), Polypropylen, Polytetrafluorethylen, Polyvinylidenchlorid, Polyvinylidenfluorid, Polytetrafluorpropylen, Polyhexafluorpropylen, Polyacrylat, Polymethylacrylat, Polymethylmethacrylat, Copolymere davon und Kombinationen, insbesondere Verbundstoffe, Mischungen oder Blends, aus wenigstens zwei der genannten Polyolefine.

Der Polyester kann insbesondere ausgewählt sein aus der Gruppe bestehend aus Polyethylenterephthalat, Polypropylenterephthalat, Polybutylenterephthalat, Copolymere davon und Kombinationen, insbesondere Verbundstoffe, Mischungen oder Blends, aus wenigstens zwei der genannten Polyester.

Das Polyamid kann insbesondere ausgewählt sein aus der Gruppe bestehend aus Polyamid 6 (Polyamid aus Caprolactam), Polyamid 46 (Polyamid aus Tetramethylendiamin und Adipinsäure), Polyamid 6.6 (Polyamid aus Hexamethylendiamin und Adipinsäure), Polyamid 69 (Polyamid aus Hexamethylendiamin und Azelainsäure), Polyamid 6/12 (Polyamid aus Hexamethylendiamin und Dodecandisäure), Polyamid 1010 (Polyamid aus 1,10-Decandiamin und Sebacinsäure), Polyamid 11 (Polyamid aus α-Aminoundecansäure), Polyamid 12 (Polyamid aus Laurinlactam), Polyamid 1212 (Polyamid aus Dodecandiamin und Dodecandisäure), Seide, Copolymere davon und Kombinationen, insbesondere Verbundstoffe, Mischungen oder Blends, aus wenigstens zwei der genannten Polyamide.

Der Polyether kann insbesondere ausgewählt sein aus der Gruppe bestehend aus Polyetherketon (PEK), Polyetheretherketon (PEEK), Polyetherketonketon (PEKK), Polyoxymethylen (POM) und Kombinationen, insbesondere Verbundstoffe, Mischungen oder Blends, aus wenigstens zwei der genannten Polyether.

Das Elastomer kann ausgewählt sein aus der Gruppe bestehend aus thermoplastisches Elastomer, insbesondere thermoplastisches Elastomer auf Olefinbasis oder Urethanbasis, thermoplastisches Polyurethan, thermoplastisches Copolyamid, thermoplastisches Polyesterelastomer, thermoplastischer Copolyester, Styrol-Blockcopolymer und Kombinationen, insbesondere Verbundstoffe, Mischungen oder Blends, aus wenigstens zwei der genannten Elastomere.

Die Keramik kann insbesondere ausgewählt sein aus der Gruppe bestehend aus Aluminiumoxidkeramik, Delta Keramik, Forte Keramik und Kombinationen, insbesondere Verbundstoffe, aus wenigstens zwei der genannten Keramiken.

Das Metall kann insbesondere ausgewählt sein aus der Gruppe bestehend aus Titan, Zirkonium, Vandium, Chrom, Molybdän, Eisen, Kobalt, Nickel, Palladium, Platin, Kupfer, Silber, Gold und Kombinationen, insbesondere Legierungen, davon. Eine geeignete Legierung stellt beispielsweise eine Kobalt-Chrom-Molybdän-Legierung dar.

In einer weiteren Ausführungsform weisen die Strukturelemente der ersten Gruppe ein anderes Material auf oder bestehen aus einem anderen Material als die Strukturelemente der zweiten Gruppe. Bezüglich geeigneter Materialien wird auf die in den vorhergehenden Abschnitten beschriebenen Materialien Bezug genommen.

Beispielsweise können die Strukturelemente der ersten Gruppe ein anderes resorbierbares Material aufweisen oder aus einem anderen resorbierbaren Material bestehen als die Strukturelemente der zweiten Gruppe. Bezüglich geeigneter Materialien wird auf die in den vorhergehenden Abschnitten beschriebenen Materialien Bezug genommen. Bezüglich geeigneter Materialien wird auf die in den vorhergehenden Abschnitten beschriebenen Materialien Bezug genommen.

Alternativ können die Strukturelemente der ersten Gruppe ein anderes nicht resorbierbares Material aufweisen oder aus einem anderen nicht resorbierbaren Material bestehen als die Strukturelemente der zweiten Gruppe. Bezüglich geeigneter Materialien wird auf die in den vorhergehenden Abschnitten beschriebenen Materialien Bezug genommen.

Bevorzugt weisen die Strukturelemente der ersten Gruppe ein resorbierbares Material auf oder bestehen aus einem resorbierbaren Material, während die Strukturelemente der zweiten Gruppe bevorzugt ein nicht resorbierbares Material aufweisen oder aus einem nicht resorbierbaren Material bestehen. Dadurch kann mit besonderem Vorteil eine Neogenese von Knochengewebe durch die Strukturelemente der ersten Gruppe induziert werden, während die Strukturelemente der zweiten Gruppe dauerhaft zur mechanischen Stabilität einer behandelten Knochenkavität beitragen können.

Alternativ können die Strukturelemente der ersten Gruppe ein nicht resorbierbares Material aufweisen oder aus einem nicht resorbierbaren Material bestehen, während die Strukturelemente der zweiten Gruppe ein resorbierbares Material aufweisen oder aus einem resorbierbaren Material bestehen können.

In einer weiteren Ausführungsform weisen die Strukturelemente der ersten Gruppe ein Additiv, insbesondere einen medizinischen Wirkstoff, einen biologischen Wirkstoff oder eine Kombination dieser Wirkstoffe, auf. In einer weiteren Ausführungsform weisen die Strukturelemente der zweiten Gruppe ein Additiv, insbesondere einen medizinischen Wirkstoff, einen biologischen Wirkstoff oder eine Kombination dieser Wirkstoffe, auf. In einer weiteren Ausführungsform weisen die Strukturelemente der ersten Gruppe und die Strukturelemente der zweiten Gruppe ein Additiv, insbesondere einen medizinischen Wirkstoff, einen biologischen Wirkstoff oder eine Kombination dieser Wirkstoffe, auf.

Der Wirkstoff ist vorzugsweise ausgewählt aus der Gruppe bestehend aus antimikrobieller, insbesondere antibiotischer, Wirkstoff, wundheilungsfördernder Wirkstoff, desinfizierender Wirkstoff, entzündungshemmender bzw. antiinflammatorischer Wirkstoff, blutgerinnungsfördernder Wirkstoff, Wachstumsfaktor wie Knochenwachstumsfaktor (osteoinduktiver Faktor), zelldifferenzierender Faktor, zelladhäsiver Faktor, zellrekrutierender Faktor, Zellrezeptor, zellbindender Faktor, Zytokin, Peptid, Strukturprotein, extrazelluläres Protein wie Collagen, Elastin, Retikulin und dergleichen, Serumprotein wie Albumin, Polysaccharid wie Hyaluronsäure, Oligonukleotid, Polynukleotid, DNA, RNA, Salze davon, Stereoisomere, insbesondere Diastereomere, davon und Mischungen aus wenigstens zwei der genannten Wirkstoffe.

Vorzugsweise handelt es sich bei dem Wirkstoff um einen Knochenwachstumsfaktor, insbesondere um ein knochenmorphogenetisches Protein (BMP). Das knochenmorphogenetische Protein ist vorzugsweise ausgewählt aus der Gruppe bestehend aus knochenmorphogenetisches Protein 1 (BMP1), knochenmorphogenetisches Protein 2 (BMP2), knochenmorphogenetisches Protein3 (BMP3), knochenmorphogenetisches Protein 3B (BMP3B)knochenmorphogenetisches Protein 4 (BMP4), knochenmorphogenetisches Protein 5 (BMP5), knochenmorphogenetisches Protein 6 (BMP6), knochenmorphogenetisches Protein 7 (BMP7), knochenmorphogenetisches Protein 8A (BMP8A), knochenmorphogenetisches Protein 8B (BMP8B), knochenmorphogenetisches Protein 10 (BMP10), knochenmorphogenetisches Protein 15 (BMP15) und Mischungen aus wenigstens zwei der genannten knochenmorphogenetischen Proteine.

In einer weiteren Ausführungsform weisen die Strukturelemente der ersten Gruppe eine Beschichtung auf, welche ein Additiv, insbesondere einen medizinischen Wirkstoff, einen biologischen Wirkstoff oder eine Kombination dieser Wirkstoffe, enthält. In einer weiteren Ausführungsform weisen die Strukturelemente der zweiten Gruppe eine Beschichtung auf, welche ein Additiv, insbesondere einen medizinischen Wirkstoff, einen biologischen Wirkstoff oder eine Kombination dieser Wirkstoffe, enthält. In einer weiteren Ausführungsform weisen die Strukturelemente der ersten Gruppe und die Strukturelemente der zweiten Gruppe eine Beschichtung auf, welche ein Additiv, insbesondere einen medizinischen Wirkstoff, einen biologischen Wirkstoff oder eine Kombination dieser Wirkstoffe, enthält. Bezüglich in Frage kommender Additive, insbesondere Wirkstoffe, wird auf die in den vorherigen Absätzen genannten Additive, insbesondere Wirkstoffe, Bezug genommen.

In einer weiteren Ausführungsform weisen die Strukturelemente der ersten Gruppe eine andere Form auf als die Strukturelemente der zweiten Gruppe. In einer alternativen Ausführungsform weisen die Strukturelemente der ersten Gruppe und die Strukturelemente der zweiten Gruppe jeweils die gleiche Form auf. Bezüglich geeigneter Formen wird auf die im folgenden Absatz genannten Formen Bezug genommen.

Beispielsweise können die Strukturelemente der ersten Gruppe und/oder die Strukturelemente der zweiten Gruppe jeweils in Form einer Kugel, eines Würfels, eines Quaders, eines Prismas, eines Spates, eines Ringes, eines Toroids oder eines Sternsvorliegen. Wie bereits erwähnt, kann es erfindungsgemäß auch vorgesehen sein, dass sich die Strukturelemente der ersten Gruppe und/oder die Strukturelemente der zweiten Gruppe untereinander hinsichtlich ihrer Form unterscheiden. Beispielsweise kann die erste Gruppe ring- und würfelförmige Strukturelemente aufweisen, während die zweite Gruppe beispielsweise quader- und würfelförmige Strukturelemente aufweisen kann.

In einer weiteren Ausführungsform handelt es sich bei dem medizinischen Produkt um ein medizinisches Produkt zur Anwendung bei der Behandlung, insbesondere zur Anwendung beim Auffüllen und/oder beim Verschluss, einer
- traumabedingten Knochenkavität,
- krankheitsbedingten, insbesondere tumorbedingten, Knochenkavität oder
- einer durch eine chirurgische Intervention oder Reintervention bedingten Knochenkavität, insbesondere einer nach einer totalen Hüft- oder Kniearthroplastik revisionsbedingten Knochenkavität.

Bei der Knochenkavität handelt es sich vorzugsweise um eine Gelenksknochenkavität, Röhrenknochenkavität oder Wirkbelkörperkavität. Besonders bevorzugt handelt es sich bei der Knochenkavität um eine Hüftgelenksknochenkavität, Kniegelenksknochenkavität, Oberschenkelknochenkavität, Schienbeinkavität, Wadenbeinkavität, Oberarmknochenkavität, Speichenkavität oder Ellenkavität.

In einer weiteren Ausführungsform liegt das medizinische Produkt, insbesondere das Flächengebilde, in Form eines Endlosbands vor.

In einer weiteren Ausführungsform liegt das medizinische Produkt, insbesondere das Flächengebilde, in konfektionierter, insbesondere abgelängter, Form vor.

Weitere Vorteile und Merkmale der Erfindung ergeben sich aus den Ansprüchen sowie aus der nachfolgenden Beschreibung bevorzugter Ausführungsformen anhand von Beispielen, Figurenbeschreibungen und der dazugehörigen Figurenbeschreibungen. Dabei können Merkmale der Erfindung jeweils für sich alleine oder in Kombination miteinander verwirklicht sein. Die nachfolgend beschriebenen Ausführungsformen dienen der weiteren Erläuterung der Erfindung, ohne diese hierauf zu beschränken.

### KURZBESCHREIBUNGEN DER FIGUREN

In den Figuren ist schematisch Folgendes gezeigt:
- Fig. 1:: zeigt eine Ausführungsform eines erfindungsgemäßen Produkts,
- Fig. 2:: einen komprimierten, insbesondere gefalteten, Zustand des in Fig. 1 dargestellten Produkts,
- Fig. 3:: einen stärker komprimierten, insbesondere gefalteten, Zustand des in Fig.1 dargestellten Produkts,
- Fig. 4:: ein Strukturelement der ersten Gruppe,
- Fig. 5: weitere Ausführungsform eines erfindungsgemäßen Produkts und
- Fig. 6:: eine weitere Ausführungsform eines erfindungsgemäßen Produkts.

### AUSFÜHRLICHE FIGURENBESCHREIBUNG

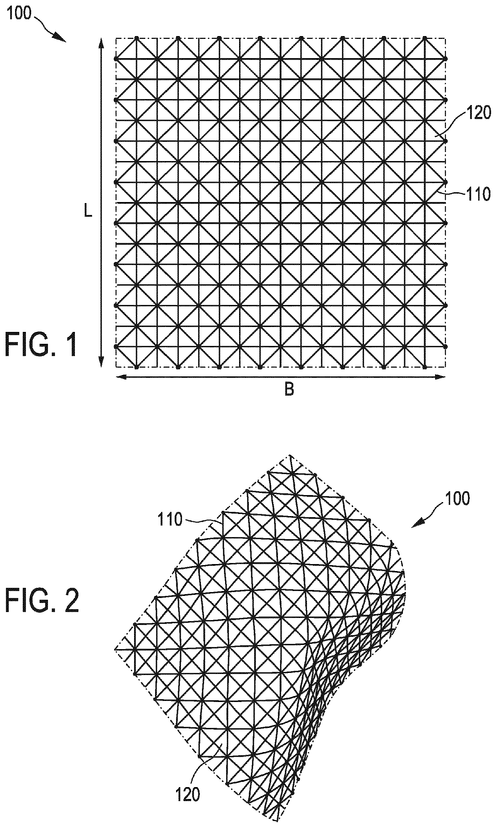

Fig. 1 zeigt schematisch eine Ausführungsform eines medizinischen Produkts 100 gemäß der vorliegenden Erfindung.

Bei dem medizinischen Produkt 100 handelt es sich vorzugsweise um ein Knochenersatz- oder Knochenfüllmaterial.

Das Produkt 100 weist eine erste Gruppe von Strukturelementen 110 und eine zweite Gruppe von Strukturelementen 120 auf. Die Strukturelemente 110 weisen dabei eine niedrigere Härte auf als die Strukturelemente 120.

Die unterschiedlichen Härten der Strukturelemente 110 und 120 bedingen insbesondere unterschiedliche Festigkeiten der Strukturelemente 110 und 120. Vorzugsweise sind die Strukturelemente 110 elastischer ausgebildet als die Strukturelemente 120 und die Strukturelemente 120 formstabiler ausgebildet als die Strukturelemente 110.

Das Produkt 100 liegt in Form eines Flächengebildes vor. Vorzugsweise sind die Länge L und die Breite B des Flächengebildes jeweils größer als die Dicke des Flächengebildes. Bei dem Flächengebilde kann es sich insbesondere um ein plattenförmiges oder teppichartiges Flächengebilde handeln.

Die Strukturelemente 110 sind jeweils länglich, bevorzugt stegförmig, ausgebildet und umranden jeweils ein Strukturelement 120. Dabei bilden die miteinander verbundenen Strukturelemente 110 Sollbiegebereiche, insbesondere Faltbereiche, des Produkts 100, entlang derer das Produkt 100 gebogen, insbesondere gefaltet, werden kann. Dadurch kann das Produkt 100 mit besonderem Vorteil an unterschiedliche Knochenkavitätsöffnungen und -formen angepasst werden. Die Sollbiegebereiche, insbesondere Faltbereiche, können, wie in Figur 1 dargestellt, in Längsrichtung, Breitenrichtung (bzw. Querrichtung) und Diagonalrichtung des Produkts 100 ausgebildet sein.

Die Strukturelemente 120 weisen jeweils die Form eines Zylinders, insbesondere Flachzylinders, mit dreieckförmigen Stirnflächen auf.

Die Strukturelemente 110 können beispielsweise aus einem resorbierbaren Material wie Polylactid, Poly-4-hydroxybutyrat oder Kollagen, oder aus einem nicht resorbierbaren Material, wie beispielsweise einem aliphatischen Polyurethan, beispielsweise aus einem unter der Bezeichnung Vasomer kommerziell erhältlichen Polyurethan, gebildet sein. Die Strukturelemente 120 können beispielsweise aus einem nichtresorbierbaren Material wie ultrahochmolekulares Polyethylen (UHMWPE), Polymethylmethacrylat, Polyamid 6.6 oder Polyetherketon gebildet sein.

Eine Biegung, insbesondere Faltung, des medizinischen Produkts 100 entlang von den Strukturelementen 110 gebildeten Sollbiegebereichen, insbesondere Faltbereichen, ist in Fig. 2 angedeutet.

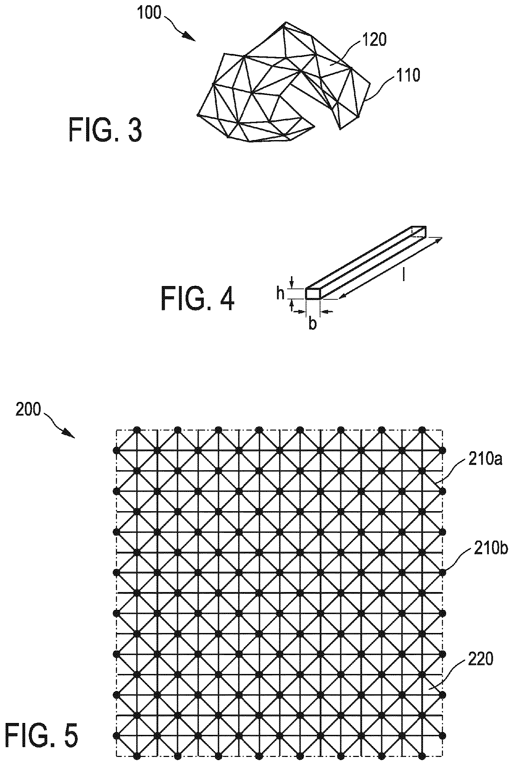

Fig. 3 zeigt schematisch das in Fig. 1 und 2 dargestellte Produkt 100 in einem stärker zusammengefalteten Zustand. In einen solchen Zustand kann das Produkt 100 durch Biegung, insbesondere Faltung, entlang mehrerer, durch die Strukturelemente gebildete Sollbiegebereiche, insbesondere Faltbereiche, überführt werden.

Fig. 4 zeigt schematisch ein Strukturelement 110 des in den Figuren 1 bis 3 dargestellten medizinischen Produkts 100. Das Strukturelement 110 ist länglich, bevorzugt stegförmig, ausgebildet. Beispielsweise kann das Strukturelement 110 in Form eines Stegs mit viereckigem, insbesondere rechteckigem, Querschnitt ausgebildet sein. Das Strukturelement 110 besitzt eine Länge I, eine Breite b sowie eine Dicke bzw. Höhe h.

Fig. 5 zeigt schematisch eine weitere Ausführungsform eines erfindungsgemäßen Produkts 200 gemäß der vorliegenden Erfindung.

Bei dem medizinischen Produkt 200 handelt es sich vorzugsweise ebenfalls um ein Knochenersatz- oder Knochenfüllmaterial.

Gegenüber dem in den Fig. 1 bis 3 dargestellten medizinischen Produkt 100 unterscheidet sich das in Fig. 5 dargestellte Produkt 200 darin, dass sich die Strukturelemente der ersten Gruppe in zwei Untergruppen einteilen lassen, nämlich in eine erste Untergruppe von Strukturelementen 210a und in eine zweite Untergruppe von Strukturelementen 210b. Dabei unterscheiden sich die Strukturelemente 210a und 210b insbesondere hinsichtlich ihrer Form. Während die Strukturelemente 210a jeweils länglich, insbesondere stegförmig, ausgebildet sind, sind die Strukturelemente 210b jeweils in Form eines Zylinders, insbesondere Flachzylinders, mit kreisförmigen Stirnflächen gestaltet. Alternativ können die Strukturelemente 210b jeweils in Form eines Zylinders, insbesondere Flachzylinders, mit polygonen, wie beispielsweise dreieckförmigen, Stirnflächen gestaltet sein (nicht dargestellt).

Die Strukturelemente 220 der zweiten Gruppe werden jeweils von drei Strukturelementen 210a sowie zwei Strukturelementen 210b umrandet.

Bezüglich weiterer Merkmale und Vorteile des in Fig. 5 dargestellten medizinischen Produkts 200 wird auf die vorangegangenen Figurenbeschreibungen Bezug genommen. Die dort in Bezug auf das Produkt 100 gemachten Ausführungen gelten sinngemäß auch für das Produkt 200.

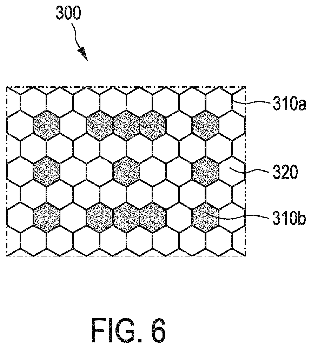

Fig. 6 zeigt schematisch eine weitere Ausführungsform eines erfindungsgemäßen Produkts 300.

Bei dem Produkt 300 lassen sich die Strukturelemente der ersten Gruppe in zwei Untergruppen einteilen, nämlich in eine erste Untergruppe von Strukturelementen 310a und in eine zweite Untergruppe von Strukturelementen 310b. Dabei unterscheiden sich die Strukturelemente 310a und 310b insbesondere hinsichtlich ihrer Form. Während die Strukturelemente 310a jeweils länglich, insbesondere stegförmig, ausgebildet sind, sind die Strukturelemente 310b jeweils in Form eines Zylinders, insbesondere Flachzylinders, mit hexagonalen Stirnflächen gestaltet.

Die Strukturelemente 320 weisen ebenfalls jeweils die Form eines Zylinders, insbesondere Flachzylinders, mit hexagonalen Stirnflächen auf.

Die Strukturelemente 320 werden von den Strukturelementen 310a und/oder den Strukturelementen 310b umrandet.

Bezüglich weiterer Merkmale und Vorteile des in Fig. 6 dargestellten medizinischen Produkts 300 wird auf die im Zusammenhang mit den Fig. 1 bis 4 gemachten Ausführungen Bezug genommen. Die dort in Bezug auf das Produkt 100 gemachten Ausführungen gelten sinngemäß auch für das Produkt 300.

Es versteht sich, dass die in den Fig. 1 bis 6 dargestellten Strukturelemente auch aus anderen Materialien gebildet sein können. Insoweit wird vollständig auf die in der allgemeinen Beschreibung genannten Materialien Bezug genommen.

## Patentansprüche

1. Medizinisches Produkt (100, 200, 300), vorzugsweise zur Anwendung bei der Behandlung, insbesondere beim Auffüllen und/oder Verschluss, einer Knochenkavität, aufweisend miteinander verbundene Strukturelemente, wobei die Strukturelemente wenigstens in zwei Gruppen von Strukturelementen einteilbar sind, nämlich wenigstens in eine erste Gruppe von Strukturelementen (110, 210a, 210b, 310a, 310b) und in eine zweite Gruppe von Strukturelementen (120, 220, 320), wobei die Strukturelemente (110, 210a, 210b, 310a, 310b) der ersten Gruppe eine niedrigere Härte aufweisen als die Strukturelemente (120, 220, 320) der zweiten Gruppe, **dadurch gekennzeichnet, dass** miteinander verbundene Strukturelemente (110, 210a, 210b, 310a, 310b) der ersten Gruppe eine Anzahl von Sollbiegebereichen des Produkts (100, 200, 300) bilden.

2. Medizinisches Produkt (100, 200, 300) nach Anspruch 1, **dadurch gekennzeichnet, dass** es sich bei dem Produkt (100, 200, 300) um ein Implantat, vorzugsweise um ein Knochenersatzmaterial, handelt.

3. Medizinisches Produkt (100, 200, 300) nach Anspruch 1 oder 2, **dadurch gekennzeichnet, dass** die Strukturelemente (110, 210a, 210b, 310a, 310b) der ersten Gruppe flexibel bzw. biegsam, insbesondere elastisch, ausgebildet sind.

4. Medizinisches Produkt (100, 200, 300) nach einem der vorhergehenden Ansprüche, **dadurch gekennzeichnet, dass** die Strukturelemente der ersten Gruppe (110, 210a, 210b, 310a, 310b) eine niedrigere Biegefestigkeit aufweisen als die Strukturelemente der zweiten Gruppe (120, 220, 320).

5. Medizinisches Produkt (100, 200, 300) nach einem der vorhergehenden Ansprüche, **dadurch gekennzeichnet, dass** die Strukturelemente der zweiten Gruppe (120, 220, 320) formstabil bzw. lasttragend ausgebildet sind.

6. Medizinisches Produkt (100, 200, 300) nach einem der vorhergehenden Ansprüche, **dadurch gekennzeichnet, dass** die Strukturelemente (110, 210a, 210b, 310a, 310b) der ersten Gruppe gleich ausgebildet oder in zwei oder mehr Untergruppen einteilbar sind, wobei die Strukturelemente jeder Untergruppe identisch zueinander und die Strukturelemente unterschiedlicher Untergruppen unterschiedlich zueinander ausgebildet sind und/oder die Strukturelemente (120, 220, 320) der zweiten Gruppe gleich ausgebildet oder in zwei oder mehr Untergruppen einteilbar sind, wobei die Strukturelemente jeder Untergruppe identisch zueinander und die Strukturelemente unterschiedlicher Untergruppen unterschiedlich zueinander ausgebildet sind.

7. Medizinisches Produkt (100, 200, 300) nach einem der vorhergehenden Ansprüche, **dadurch gekennzeichnet, dass** es sich bei den Strukturelementen (110, 210a, 210b, 310a, 310b) der ersten Gruppe um längliche Strukturelemente, d.h. um Strukturelemente mit einem Längen-Breiten-Verhältnis oder Längen-Durchmesser-Verhältnis größer 1 handelt und/oder das Produkt (100, 200, 300) ein aus den Strukturelementen der ersten und zweiten Gruppe aufgebautes Flächengebilde, insbesondere plattenförmige Flächengebilde, aufweist, dessen Länge und Breite jeweils größer ist als dessen Dicke bzw. Höhe und/oder die Strukturelemente der ersten und/oder zweiten Gruppe Löcher oder Öffnungen aufweisen.

8. Medizinisches Produkt (100, 200, 300) nach Anspruch 7, **dadurch gekennzeichnet, dass** die miteinander verbundenen Strukturelemente (110, 210a, 210b, 310a, 310b) der ersten Gruppe eine Anzahl von Sollbiegebereichen des Flächengebildes bilden.

9. Medizinisches Produkt (100, 200, 300) nach Anspruch 8, **dadurch gekennzeichnet, dass** die Sollbiegebereiche jeweils wenigstens ein, insbesondere nur ein, Strukturelement (120, 220, 320) der zweiten Gruppe umranden.

10. Medizinisches Produkt (100, 200, 300) nach Anspruch 9, **dadurch gekennzeichnet, dass** die Sollbiegebereiche das wenigstens eine Strukturelement (120, 220, 320) der zweiten Gruppe eckenlos, insbesondere kreisringförmig, umranden.

11. Medizinisches Produkt (100, 200, 300) nach Anspruch 9, **dadurch gekennzeichnet, dass** die Sollbiegebereiche das wenigstens eine Strukturelement (120, 220, 320) der zweiten Gruppe polygon, insbesondere dreieck-, viereck-, fünfeck- und/oder sechseckförmig, umranden.

12. Medizinisches Produkt (100, 200, 300) nach einem der Ansprüche 8 oder 11, **dadurch gekennzeichnet, dass** die Sollbiegebereiche jeweils reihenförmig, insbesondere in Form von zueinander parallel angeordneten Reihen, und/oder kurvenförmig, bogenförmig oder mäanderförmig wie sinusförmig ausgebildet sind.

13. Medizinisches Produkt (100, 200, 300) nach einem der Ansprüche 8 bis 12, **dadurch gekennzeichnet, dass** es sich bei den Sollbiegebereichen um Faltbereiche des Produkts (100, 200, 300) handelt.

14. Medizinisches Produkt (100, 200, 300) nach einem der Ansprüche 8 bis 13, **dadurch gekennzeichnet, dass** die Sollbiegebereiche wenigstens zum Teil über ein oder mehrere Strukturelemente (120, 220, 320) der zweiten Gruppe miteinander verbunden sind.

15. Medizinisches Produkt (100, 200, 300) nach einem der vorhergehenden Ansprüche, **dadurch gekennzeichnet, dass** die Strukturelemente der ersten und/oder zweiten Gruppe ein resorbierbares Material aufweisen oder aus einem solchen Material bestehen, wobei das Material vorzugsweise ausgewählt ist aus der Gruppe bestehend aus Polyhydroxyalkanoat, Protein wie extrazelluläres Protein, Polysaccharid wie Cellulosederivat oder Mucopolysaccharid, Polyhydroxyalkanoat, Stereoisomere, insbesondere Diastereomere, davon, Salze davon, Copolymere davon und Kombinationen, insbesondere Verbundstoffe, Mischungen oder Blends, aus wenigstens zwei der genannten Materialien und/oder die Strukturelemente der ersten und/oder zweiten Gruppe ein nicht resorbierbares Material aufweisen oder aus einem solchen Material bestehen, wobei das Material vorzugsweise ausgewählt ist aus der Gruppe bestehend aus Polymer wie Polyolefin, Polyester oder Polyamid, Metall, Keramik und Kombinationen, insbesondere Verbundstoffe, Mischungen oder Blends, aus wenigstens zwei der genannten Materialien.

## Claims

1. Medical product (100, 200, 300), preferably for use in the treatment, more particularly in the filling up and/or closure, of a bone cavity, having structural elements connected to one another, wherein the structural elements are dividable at least into two groups of structural elements, namely at least into a first group of structural elements (110, 210a, 210b, 310a, 310b) and into a second group of structural elements (120, 220, 320), wherein the structural elements (110, 210a, 210b, 310a, 310b) of the first group have a lower hardness than the structural elements (120, 220, 320) of the second group, **characterized in that** structural elements (110, 210a, 210b, 310a, 310b) of the first group that are connected to one another form a number of intended bending regions of the product (100, 200, 300).

2. Medical product (100, 200, 300) according to claim 1, **characterized in that** the product (100, 200, 300) is an implant, preferably a bone substitute material.

3. Medical product (100, 200, 300) according to claim 1 or 2, **characterized in that** the structural elements (110, 210a, 210b, 310a, 310b) of the first group are flexible or bendy, more particularly elastic.

4. Medical product (100, 200, 300) according to any of the preceding claims, **characterized in that** the structural elements of the first group (110, 210a, 210b, 310a, 310b) have a lower bending strength than the structural elements of the second group (120, 220, 320).

5. Medical product (100, 200, 300) according to any of the preceding claims, **characterized in that** the structural elements of the second group (120, 220, 320) are dimensionally stable or load-bearing.

6. Medical product (100, 200, 300) according to any of the preceding claims, **characterized in that** the structural elements (110, 210a, 210b, 310a, 310b) of the first group are identical or dividable into two or more subgroups, the structural elements of each subgroup being identical to one another and the structural elements of different subgroups being different from one another and/or the structural elements (120, 220, 320) of the second group are identical or dividable into two or more subgroups, the structural elements of each subgroup being identical to one another and the structural elements of different subgroups being different from one another.

7. Medical product (100, 200, 300) according to any of the preceding claims, **characterized in that** the structural elements (110, 210a, 210b, 310a, 310b) of the first group are elongated structural elements, i.e., structural elements having a length-to-width ratio or length-to-diameter ratio greater than 1 and/or the product (100, 200, 300) has a planar structure constructed from the structural elements of the first and second groups, in particular sheet-shaped planar structure, the length and width of which planar structure is in each case larger than the thickness or height of said planar structure, and/or the structural elements of the first and/or second group have holes or openings.

8. Medical product (100, 200, 300) according to claim 7, **characterized in that** the structural elements (110, 210a, 210b, 310a, 310b) of the first group that are connected to one another form a number of intended bending regions of the planar structure.

9. Medical product (100, 200, 300) according to claim 8, **characterized in that** the intended bending regions surround in each case at least one, more particularly just one, structural element (120, 220, 320) of the second group.

10. Medical product (100, 200, 300) according to claim 9, **characterized in that** the intended bending regions surround the at least one structural element (120, 220, 320) of the second group in a cornerless manner, more particularly in the shape of a circular ring.

11. Medical product (100, 200, 300) according to claim 9, **characterized in that** the intended bending regions surround the at least one structural element (120, 220, 320) of the second group in a polygonal manner, more particularly in the shape of a triangle, in the shape of a quadrangle, in the shape of a pentagon and/or in the shape of a hexagon.

12. Medical product (100, 200, 300) according to any of claims 8 to 11, **characterized in that** the intended bending regions are in each case in rows, more particularly in the form of rows arranged in parallel to one another, and/or are curved, arched or meandering, such as sinusoidal.

13. Medical product (100, 200, 300) according to any of claims 8 to 12, **characterized in that** the intended bending regions are folding regions of the product (100, 200, 300).

14. Medical product (100, 200, 300) according to any of claims 8 to 13, **characterized in that** the intended bending regions are connected to one another at least in part via one or more structural elements (120, 220, 320) of the second group.

15. Medical product (100, 200, 300) according to any of the preceding claims, **characterized in that** the structural elements of the first and/or second group comprise a resorbable material or consist of such a material, the material preferably being selected from the group consisting of polyhydroxyalkanoate, protein, such as extracellular protein, polysaccharide, such as cellulose derivative or mucopolysaccharide, polyhydroxyalkanoate, stereoisomers, more particularly diastereomers, thereof, salts thereof, copolymers thereof and combinations, more particularly composites, mixtures or blends, of at least two of the stated materials, and/or the structural elements of the first and/or second group comprise a non-resorbable material or consist of such a material, the material preferably being selected from the group consisting of polymer, such as polyolefin, polyester or polyamide, metal, ceramic and combinations, more particularly composites, mixtures or blends, of at least two of the stated materials.

## Revendications

1. Produit médical (100, 200, 300), de préférence destiné à une utilisation dans le traitement, en particulier dans le remplissage et/ou la fermeture, d'une cavité osseuse, comprenant des éléments structuraux reliés les uns aux autres, les éléments structuraux pouvant être divisés en au moins deux groupes d'éléments structuraux, à savoir au moins en un premier groupe d'éléments structuraux (110, 210a, 210b, 310a, 310b) et en un deuxième groupe d'éléments structuraux (120, 220, 320), les éléments structuraux (110, 210a, 210b, 310a, 310b) du premier groupe présentant une dureté inférieure à celle des éléments structuraux (120, 220, 320) du deuxième groupe, **caractérisé en ce que** des éléments structuraux reliés les uns aux autres (110, 210a, 210b, 310a, 310b) du premier groupe forment un nombre de zones de fléchissement privilégié du produit (100, 200, 300).

2. Produit médical (100, 200, 300) selon la revendication 1, **caractérisé en ce que** le produit (100, 200, 300) consiste en un implant, de préférence en un matériau de substitution osseuse.

3. Produit médical (100, 200, 300) selon la revendication 1 ou 2, **caractérisé en ce que** les éléments structuraux (110, 210a, 210b, 310a, 310b) du premier groupe sont configurés sous forme flexible ou souple, en particulier élastique.

4. Produit médical (100, 200, 300) selon l'une quelconque des revendications précédentes, **caractérisé en ce que** les éléments structuraux du premier groupe (110, 210a, 210b, 310a, 310b) présentent une résistance à la flexion plus faible que les éléments structuraux du deuxième groupe (120, 220, 320).

5. Produit médical (100, 200, 300) selon l'une quelconque des revendications précédentes, **caractérisé en ce que** les éléments structuraux du deuxième groupe (120, 220, 320) sont configurés sous forme dimensionnellement stable ou porteuse de charge.

6. Produit médical (100, 200, 300) selon l'une quelconque des revendications précédentes, **caractérisé en ce que** les éléments structuraux (110, 210a, 210b, 310a, 310b) du premier groupe sont configurés de la même manière ou peuvent être divisés en deux sous-groupes ou plus, les éléments structuraux de chaque sous-groupe étant configurés sous forme identique les uns aux autres et les éléments structuraux de différents sous-groupes étant configurés sous forme différente les uns des autres, et/ou les éléments structuraux (120, 220, 320) du deuxième groupe sont configurés de la même manière ou peuvent être divisés en deux sous-groupes ou plus, les éléments structuraux de chaque sous-groupe étant configurés sous forme identique les uns aux autres et les éléments structuraux de différents sous-groupes étant configurés sous forme différente les uns des autres.

7. Produit médical (100, 200, 300) selon l'une quelconque des revendications précédentes, **caractérisé en ce que** les éléments structuraux (110, 210a, 210b, 310a, 310b) du premier groupe consistent en des éléments structuraux allongés, c.-à-d. en des éléments structuraux ayant un rapport longueur-largeur ou un rapport longueur-diamètre supérieur à 1, et/ou le produit (100, 200, 300) présente une structure plane construite à partir des éléments structuraux du premier et deuxième groupe, en particulier une structure plane en forme de plaque, dont la longueur et la largeur sont chacune plus grandes que son épaisseur ou sa hauteur, et/ou les éléments structuraux du premier et/ou deuxième groupe comprennent des trous ou des ouvertures.

8. Produit médical (100, 200, 300) selon la revendication 7, **caractérisé en ce que** les éléments structuraux reliés les uns aux autres (110, 210a, 210b, 310a, 310b) du premier groupe forment un nombre de zones de fléchissement privilégié de la structure plane.

9. Produit médical (100, 200, 300) selon la revendication 8, **caractérisé en ce que** les zones de fléchissement privilégié bordent chacune au moins un, en particulier uniquement un, élément structural (120, 220, 320) du deuxième groupe.

10. Produit médical (100, 200, 300) selon la revendication 9, **caractérisé en ce que** les zones de fléchissement privilégié bordent l'au moins un élément structural (120, 220, 320) du deuxième groupe sans angles, en particulier en forme d'anneau circulaire.

11. Produit médical (100, 200, 300) selon la revendication 9, **caractérisé en ce que** les zones de fléchissement privilégié bordent l'au moins un élément structural (120, 220, 320) du deuxième groupe sous forme polygonale, notamment triangulaire, quadrangulaire, pentagonale et/ou hexagonale.

12. Produit médical (100, 200, 300) selon l'une quelconque des revendications 8 ou 11, **caractérisé en ce que** les zones de fléchissement privilégié sont chacune configurées sous forme de ligne, en particulier sous forme de lignes agencées parallèlement les unes aux autres, et/ou sous forme incurvée, sous forme arquée ou sous forme de méandres, telle que sinusoïdale.

13. Produit médical (100, 200, 300) selon l'une quelconque des revendications 8 à 12, **caractérisé en ce que** les zones de fléchissement privilégié consistent en des zones de pliage du produit (100, 200, 300).

14. Produit médical (100, 200, 300) selon l'une quelconque des revendications 8 à 13, **caractérisé en ce que** les zones de fléchissement privilégié sont au moins en partie reliées les unes aux autres par l'intermédiaire d'un ou de plusieurs éléments structuraux (120, 220, 320) du deuxième groupe.

15. Produit médical (100, 200, 300) selon l'une quelconque des revendications précédentes, **caractérisé en ce que** les éléments structuraux du premier et/ou deuxième groupe comprennent un matériau résorbable ou sont constitués d'un tel matériau, le matériau étant de préférence choisi dans le groupe constitué par un polyhydroxyalcanoate, une protéine telle qu'une protéine extracellulaire, un polysaccharide tel qu'un dérivé de cellulose ou un mucopolysaccharide, un polyhydroxyalcanoate, des stéréoisomères, en particulier des diastéréomères, de ceux-ci, des sels de ceux-ci, des copolymères de ceux-ci et des combinaisons, en particulier des matières composites, des mélanges ou des mélanges homogènes, d'au moins deux des matériaux mentionnés, et/ou les éléments structuraux du premier et/ou deuxième groupe comprennent un matériau non résorbable ou sont constitués d'un tel matériau, le matériau étant de préférence choisi dans le groupe constitué par un polymère tel qu'une polyoléfine, un polyester ou un polyamide, un métal, une céramique et des combinaisons, en particulier des matières composites, des mélanges ou des mélanges homogènes, d'au moins deux des matériaux mentionnés.
